(19) Europäisches Patentamt
European Patent Office
Office européen des brevets

(11) EP 0 530 850 B1

(12) EUROPEAN PATENT SPECIFICATION

(45) Date of publication and mention of the grant of the patent: **09.09.1998 Bulletin 1998/37**

(51) Int Cl.[6]: **C12P 19/20**, C07H 15/203, C12Q 1/40, C12P 19/00

(21) Application number: **92116958.7**

(22) Date of filing: **10.07.1987**

# (54) Process for producing a modified oligosaccharide

Verfahren zur Herstellung eines modifizierten Oligosaccharids

Procédé pour la production d'une oligosaccharide modifiée

(84) Designated Contracting States:
**AT BE CH DE ES FR GB GR IT LI LU NL SE**

(30) Priority: **11.07.1986 JP 163054/86**
**09.01.1987 JP 2730/87**
**26.01.1987 JP 15776/87**

(43) Date of publication of application:
**10.03.1993 Bulletin 1993/10**

(62) Document number(s) of the earlier application(s) in accordance with Art. 76 EPC:
**87110023.6 / 0 252 525**

(73) Proprietor: **WAKO PURE CHEMICAL INDUSTRIES, LTD.**
**Higashi-ku Osaka (JP)**

(72) Inventors:
- **Ikenaka, Tokuji**
  **Sakai-shi (JP)**
- **Omichi, Kaoru**
  **Toyonaka-shi (JP)**
- **Satomura, Shinji**
  **Yodogawa-ku, Osaka (JP)**
- **Natsuka, Yuko**
  **Ann Arbor, Michigan 48105 (US)**

(74) Representative: **Baillie, Iain Cameron**
**Langner Parry,**
**52-54 High Holborn**
**London WC1V 6RR (GB)**

(56) References cited:
**EP-A- 0 171 960** **EP-A- 0 173 255**

- **CHEMICAL ABSTRACTS, vol. 103, no. 1, 8 July 1985 Columbus, Ohio, US; abstract no. 2743p, K.OMICHI ET AL. 'preparation of non-reducing-end Substituted p-nitrophenyl alpha-Maltopentaoside (FG5P) as a Substrate for a Coupled Enzymatic Assay for alpha Amylases' page 256;**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 530 850 B1

## Description

This invention relates to a process for producing a modified oligosaccharide.

Heretofore, various processes for determining the activity of α-amylase using a modified oligosaccharide as a substrate have been proposed. For example, U.S. Patent No. 4,649,108 to Blair discloses a method of measuring be amount of α-amylase in liquid sample comprising the steps of providing an oligosaccharide substrate for α-amylase, said substrate containing at least 3 glucose units, the reducing-end glucose residue (unit) being bonded, via a bond cleavable by α- or β-glucosidase, to a label which exhibits an optically measurable change upon cleavage of said bond, and the non-reducing-end (terminal) glucose residue being bonded to a blocking group which inhibits cleavage by exo-enzymes of the bond between said non-reducing-end glucose redidue and the adjacent glucose residue; contacting said sample with said oligosaccharide substrate and with a first added exo-enzyme capable of cleaving the bond between said reducing-end glucose residue and said label, and a second added exo-enzyme capable of cleaving bonds between glucose units, to form a mixture comprising said substrate, said first exo-enzyme, and said second exo-enzyme; and measuring said optically measurable change. According to said U.S. patent, the blocking groups can be carboxylic acid esters, phosphate esters, sulfonate esters, ethers (such as benzyl, silyl, and triphenylmethyl), monosaccharides other than α-1,4 linked glucose, and acetal or ketal blocking groups such as benzylidene (column 4 lines 37-67). But the ester blocking groups are unstable since they are easily hydrolyzed in an aqueous solution. Further, when the size of the blocking group becomes larger as in the case of toluenesulfonyl, methanesulfonyl, silyl or triphenylmethyl, the solubility in water is undesirably lowered. In addition, the introduction of a blocking group into the non-reducing-end glucose residue has many problems, and the ester and ether groups as mentioned above cannot be introduced into the non-reducing-end glucose residue by the process disclosed at from column 4 line 63 to column 7 line 58 of said U.S. patent. Therefore, according to said U.S. patent, preferably the substrate has eight or fewer glucose units, and most preferably has six or seven, and preferred blocking substituents are acetals or ketals, e.g. benzylidene (column 2 lines 4-7).

When the most preferred oligosaccharide according to said U.S. patent, that is, an oligosaccharide having 6 or 7 glucose units and benzylidene as the blocking group, is used as a substrate for measuring α-amylase, there are many problems in that the hydrolysis rate by α-amylase is slow, various kinds of hydrolyzed products are produced due to many positions to be hydrolyzed, the reaction for liberating the color producing group becomes many and complicated due to many hydrolyzed products, the amount of coupling enzymes to be used becomes larger, and thus the reliability for the measurement becomes insufficient. This invention provides processes for the production of novel modified oligosaccharides which are useful in improved processes for determining α-amylase activity in a sample.

In the disclosure of K.OMICHI ET AL. preparation of non-reducing-end Substituted p-nitrophenyl alpha-Maltopentaoside (FG5P) as a Substrate for a Coupled Enzymatic Assay for alpha Amylases' ordinary oligosaccharides for example Fg6 (see for example page 978 right hand column lines 2-6 of the reference) are used as reactants [J. Biochem. 97, 977 (1985)].

EP-A-0,173,255 discloses oligosaccharide derivatives and their use as a substrate for measuring alpha amylase activity. It is disclosed that starting materials for the preparation of the oligosaccharide derivatives of the reference can be ordinary polysaccharides such as dextrin and amylose.

## SUMMARY OF THE INVENTION

This invention provides a process for producing an oligosaccharide having a special substituent at the non-reducing-end glucose residue, which comprises reacting a modified cyclodextrin with cyclomaltodextrin-gluconotransferase in the present of a specified acceptor, and then reacting with glucoamylase or α-glucosidase.

This invention further provides a process for producing an oligosaccharide having a special substituent at the non-reducing-end glucose residue which comprises reacting an oligosaccharide having a substituent which exhibits an optically measurable change upon cleavage at the reducing-end glucose residue with an aldehyde, a ketone, an acetal or a ketal to form a 4,6-0-cyclic acetal or a 4,6-0-cyclic ketal at the non-reducing-end glucose residue and reducing said 4,6-0-cyclic acetal or ketal.

## BRIEF DESCRIPTION OF THE DRAWINGS

Figs. 1 and 2 are FAB mass spectra of the oligosaccharides obtained in Examples 3 and 5 (apparatus : Jeol HX-100), respectively.

Fig. 3 is an IR chart of the oligosaccharide obtained in Example 6.

Figs. 4 and 5 are $^1$H-NMR charts of the oligosaccharides obtained in Examples 6 and 8, respectively.

Figs. 6 and 7 are calibration curves obtained in Examples 12 and 14, respectively.

DESCRIPTION OF THE PREFERRED EMBODIMENTS

The modified oligosaccharide compounds of the invention can be produced by reacting a modified cyclodextrin with cyclomaltodextrin-glucanotransferase in the presence of a specified acceptor, and then reacting with glucoamylase or α-glucosidase.

These compounds can be represented by the formula :

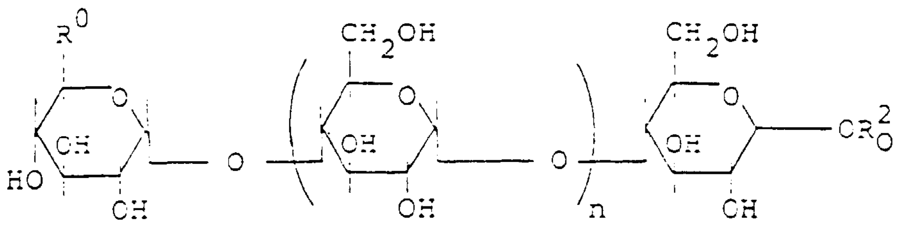

I

wherein

$R^0$ is an alkoxymethyl group, a benzyloxymethyl group, an aminomethyl group, a carboxyl group, a halomethyl group, a pyridylaminomethyl group, or a carboxymethoxymethyl group,

$R^2_O$ is glucitol residue or a group of the formula:

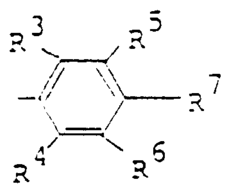 , 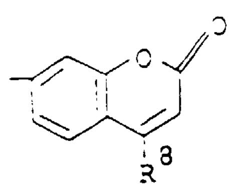 or 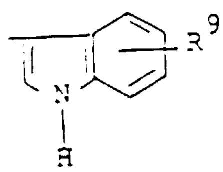

in which $R^3$ through $R^6$ are independently hydrogen, a lower alkyl group, a lower alkoxy group, a nitro group, a carboxyl group, a sulfonic acid group or a halogen atom, and $R^3$ and $R^5$, and/or $R^4$ and $R^6$, may be bonded to form an aromatic ring; $R^7$ is hydrogen, a lower alkoxy group, a halogen atom, or a nitro group; $R^8$ is hydrogen, a methyl group, or a trifluoromethyl group; and $R^9$ is hydrogen or a halogen atom; and n is an integer of 2 to 5,

As the modified cyclodextrin, there can preferably be used those having one modified glucose residue per molecule, and may contain those having two or more modified glucose residues per molecule.

As the modified glucose residue, there can be used substituted glucose wherein the hydroxyl group in the glucose is replaced by a substituent which can emit fluorescence, e.g. a 2-pyridylamino group or a 3-pyridylamino group; a substituent which can absorb UV light, e.g. an anilino group, a methylanilino group, a hydroxyanilino group, or a carboxyphenylamino group; an alkoxy group such as a methoxy group, or an ethoxy group; a substituted alkoxy group such as a carboxy-methoxy group, a hydroxyethoxy group, a benzyloxy group, a phenetyloxy group or a pyridylmethyloxy group; a halogen atom such as chlorine, or bromine; a hydrazono group, a phenylhydrazono group or an amino group; or there can be used glucuronic acid wherein the $-CH_2OH$ group at the $C_6$ position of glucose is replaced by a -COOH group.

As the acceptor, there can be used an acceptor selected from derivatives of glucose, maltose and maltotriose wherein the hydroxyl group at the $c_1$ position of the acceptor is replaced by a substituent represented by the formula: $-OR^2_O$ ,wherein $R^2_O$ is as defined above e.g. p-nitrophenyl, phenyl, umbelliferyl, naphtyl. When maltotetraose or higher oligosaccharides are used, the reaction is slow and causes side reactions undesirably. On the other hand, when the glucose chain becomes longer, the glucose chain of the major product in the initial reaction also becomes longer. Therefore, the glucose chain length of 1 to 3 is properly selected and used as the acceptor depending on the glucose chain length of the desired product. When a glucose derivative is used as the acceptor, the position of substituent is the $C_1$ position, irrespective of α-substitution or β-substitution.

As the substituent of the hydroxyl group at the $C_1$ position of the modified glucose residue of the acceptor, there is used the group represented by the formula: $-OR^2{}_O$ wherein $R^2{}_O$ is as defined above.

Concrete examples of the $-OR^2{}_O$ groups are a p-nitrophenoxy group, a m-nitrophenoxy group, an o-chlorophenoxy

group, a p-chlorophenoxy group, a 2,6-dichlorophenoxy group, an o-methoxyphenoxy group, a p-methoxyphenoxygroup, an o-methylphenoxy group, an o-carboxyphenoxy group, an o-sulfophenoxy group, a 1-naphthoxygroup, a 2-sulfo-1-naphthoxy group, a 2-carboxy-1-naphthoxy group, an umbelliferyl group, a 4-methyl-umbelliferyl group, an indoxyl group, or a glucitol residue.

The cyclomaltodextrin-glucanotransferase (here-imafter referred to as "CGTase") is not particularly limited to its origin and source. There can be used those derived from Bacillus macerans, Bacillus megaterium, Klebsiella pneumonie, alcaligenous, etc.

The modified cyclodextrin can easily be obtained by using α-, β- or γ-cyclodextrin as a starting material and reacting with various reactants for introducing the desired modifying groups thereinto according to processes for producing various modified glucoses described in, for example, Method in Carbohydrate Chemistry I (1962) to V (1965), published by Academic Press. The selection of α-, β - or γ -cyclodextrin can be determined depending on the glucose chain length of the desired modified oligosaccharide derivative and its maximum yield.

The pH of the reaction of the modified cyclodextrin with CGTase in the presence of an accptor changes slightly depending on the origin of CGTase, but usually 6 to S. Any buffering agent which does not inhibit the enzymatic reaction can be used for maintaining the suitable pH. Examples of the buffering agents are Good's buffer, ammonium acetate buffer, carbonate buffer, and phosphate buffer.

The modified cyclodextrin is used in a concentration of preferably 1 to 50 mmol/l and the acceptor is used in a concentraiton of 1 mmol/l or more to the solubility limit.

The acceptor is preferably used in an amount of 5 moles or more per mole of the modified cyclodextrin.

The CGTase is preferably used in an amount of 50 to 5000 U/ml. The reaction is preferably carried out at 20 to 50°C. After the enzymatic reaction, CGTase is deactivated by heating, for example at 90°C or higher for 10 minutes or more, or by changing the pH, for example pH of 4.0 or less.

The reaction using glucoamylase or α-glucosidase can preferably be carried out at most suitable pH, for example pH 4 to 6, and using glucoamylase or α-glucosidase in an amount of preferably 5 to 100 U/ml.

Alternatively, the modified oligosaccharide compound of the formula (II) can be produced by reacting an oligosaccharide having a substituent which exhibits an optically measurable change upon cleavage at the reducing-end glucose residue with an aldehyde, a ketone, an acetal or a ketal to form a 4,6-0-cyclic acetal or a 4,6-0-cyclic ketal at the non-reducing end glucose residue, and reducing said 4,6-0-cyclic acetal or ketal.

The compounds of formula II are represented as follows:

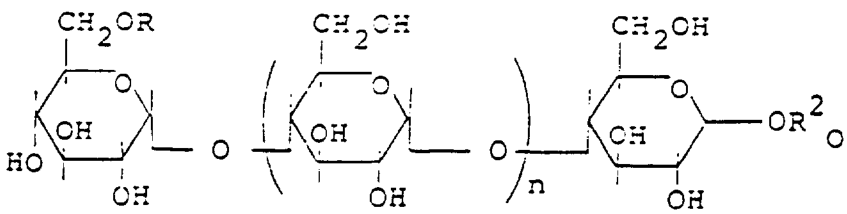 II

wherein R is a benzyl group, a substituted benzyl group (wherein the substituent group is an alkyl group having 1 to 4 carbon atoms, an alkoxy group having 1 to 4 carbon atoms, an alkyl-substituted amino group, a carboxyl group, a nitro group, or a halogen atom such as chlorine, bromine or iodine), a 2-, 3- or 4-pyridylmethyl group, a straight-or branched-chain or cyclic alkyl group having 1 to 6 carbon atoms, or an alkenyl group having 1 to 6 carbon atoms $R^2_O$ is as defined above; and n is an integer of 2 to 5.

The oligosaccharide used as a starting material can be represented by the formula:

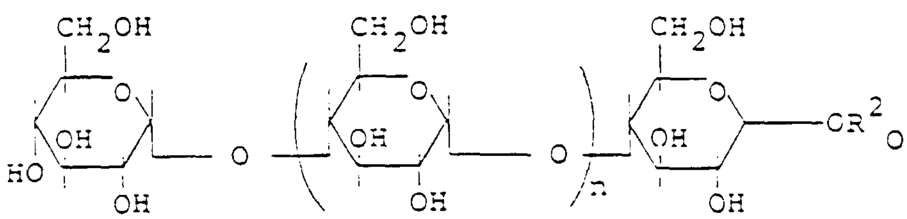 (VIII)

wherein $R^2_O$ and n are as defined above.

First, the oligosaccharide derivative of the formula (VIII) is reacted with an aldehyde, a ketone, an acetal or a ketal to form a cyclic acetal between the $C_4$ and $C_6$ positions of the non-reducing-end-glucose residue. The oligosaccharide derivative of the formula (VIII) is available commercially or can be synthesized by a process described in, for example, Japanese Patent Unexamined Publication No. 54-51892.

As the aldehyde, there can be used an aromatic aldehyde such as benzaldehyde, or tolylaldehyde; aliphatic aldehydes such as formaldehyde, acetaldehyde, propionaldehyde, or butylaldehyde.

As the ketone, there can be used acetone, or methyl ethyl ketone.

As the acetal, there can be used methylal, benzaldehyde dimethylacetal, acetaldehyde dimethylacetal, or acetaldehyde diethylacetal.

As the ketal, there can be used methyl ethyl ketone ethyleneketal or cyclohexanone dimethylketal.

Further, there can also be used any aldehydes, ketones, acetals and ketals which can form a cyclic acetar or cyclic ketal between the $C_4$ and $C_6$ positions of the non-reducing end glucose residue of the oligosaccharide derivative of the formula (II).

The aldehyde, ketone, acetal or ketal is used in an amount of preferably 1 to 100 moles per mole of the oligosaccharide derivative (VIII).

The reaction is preferably carried out in the presence of a Lewis acid catalyst such as p-toluenesulfonic acid, or zinc chloride, in a suitable solvent such as N,N-dimethylformamide (DMF), at from room temperature to a reflux temperature for 0.5 to 12 hours.

The resulting intermediate having the cyclic acetal is usually subjected to modification of other hydroxyl groups by acylation, followed by a reduction step.

The acylation can be carried out by a conventional process, for example, in the presence of a base such as pyridine and using as an acylating agent such as acetic anhydride, acetylchloride, or benzoylchloride.

The acylation step is not essential but is preferable to enhance the solubility in a reaction solvent used in the next step (the reduction step).

The reduction step is usually carried out by using a reducing agent in the presence of an acid catalyst such as aluminum chloride, boron trifluoride, zinc chloride, p-toluenesulfonic acid, methanesulfonic acid, hydrogen chloride gas (or blown into ether)., in a suitable solvent (e.g. tetrahydrofuran (THF) usually at 0 - 50°C for several tens of minutes to several hours.

As the reducing agent, there can be used sodium cyanoborohydride, lithium aluminiumhydride, pyridineborane, dimethylamineborane, trimethylamineborane, t-butylamine-borane, or diborane. The reudcing agent is preferably used in an amount of 1 to 1000 moles per mole of the oligosaccharide derivative (VIII).

Tine acid catalyst is preferably used in an amount of 0.5 to 5 moles per mole of the reducing agent.

When the acyl protction is conducted, deacylation is conducted by a conventional process, for example, by treating with a 0.01 to 1.0 N sodium methoxide-methanol solution for up to several tens of hours at from room temperature to slightly elevated temperatures to easily give the modified oligosaccharide of the formula (II). Individual after-treatments after individual stets can be carried out by conventional processes. The final product can be purified by a conventional process such as column chromatography,

Since the α-1,4 bond of the oligosaccharide is subjected to acid hydrolysis under acidic conditions, or methanolysis under anhydrous methanol-HCl conditions, and the stability of the glycosidic bond (the bonding between the reducing end glucose residue and the -$OR^2_O$ group) under acidic conditions was unknown, it was unknown whether the modified olgosaccharide of the formula (II) derived from the oligosaccharide of the formula (VIII) could withstand such reducing treatment. But unexpectedly, the present inventors have found for the first time that ring-opening of the cyclic acetal portion was possible and accomplished the present invention.

In the formula (II), the alkyl group in the substituted benzyl group includes a methyl group, an ethyl group, a propyl group, and a butyl group; the alkoxy group in the substituted benzyl group includes a methoxy group, an ethoxy group, a propoxy group, and a butoxy group; the alkyl-substituted amino group includes a dimethylamino group, a diethylamino group, and an N-ethyl-N-(β-hydroxyethyl) amino group; the straight- or branched-chain or cyclic clkyl group having 1 to 6 carbon atoms includes a methyl group, an ethyl group, an iso-propyl group, a n-butyl group, a tert-butyl group, a n-pentyl group, an iso-amyl group, a n-hexyl group, a cyclopentyl group, a cyclohexyl group, etc.; and the alkenyl group having 1 to 6 carbon atoms includes an ethenyl group, a 2-propenyl group, a 2butenyl group.

The group of the formula: -$OR^2_O$ in the formula (II) is bonded to the reducing end glucose residue and can be hydrolyzed by the action of glucoamylase-[E.C.3.2.1.3], α-glucosidase[E.C.3.2.1.20.], β-glucosidase [E.C.3.2.1.21.], isomaltase-[E.C.3.2.1.10] or β-amylase[E.C.3.2.1.2.], to form nitrophenols which have absorptions by themselves at visible light range, to finally form dyes by coupling with couplers by the action of oxidases such as catechol oxidase, laccase, tyrosinase and monophenol oxidase, or to finally form dyes by coupling with couplers by the action of oxidants.

Examples of the group of the formula: -$OR^2_O$ in the formula II (many of which are also mentioned above) are a p-nitrophenoxy group, a m-nitrophenoxy group, an o-chlorophenoxy group, a p-chlorophenoxy group, a 2,6 -dichlorophenoxy group, a 2-chloro-4-nitrophenoxy group, an o-methoxyphenoxy group, a p-methoxyphenoxy group, an o-methylphenoxy group, an carboxyphenoxy group, an o-sulfophenoxy group, a 1-naphtoxy group, a 2-sulfo-1-naphtoxy group, a 2-carboxy-1-naphtoxy group, etc.; an umbelliferyl group, a 4-methylumbelliferyl group, a 4-trifluromethylumbelliferyl group, etc.; an indoxyl group, 5-bromoindoxyl group, 4-chloro-3-bromoindoxyl group, a glucitol residue.

The compounds of the formulae (I) and (II) can be used as substrates for determining the activity of α-amylase.

A part of the modified oligosaccharide of this inventions can effectively be used as a substrate for a fractional measuring method of α-amylase derived from the salivary glands and α-amylase derived from pancreas, that is, a fractional measuring method of α-amylase isozymes wherein decomposed products produced by hydrolysis of α-amylase are reacted with two or more coupling enzymes having different substrate specificities and the produced products are measured to conduct fractional measurements of α-amylase derived from human pancreas and α-amylase derived from human salivary glands.

This invention is illustrated by way of the following Examples.

Example 1

Synthesis of p-nitrophenyl O-6-deoxy-6-[(2-pyridyl)amino]-α-D-glucopyranosyl-(1→4)-O-α-D-glucopyranosyl-(1→4)-C-α-D-glucopyranosyl-(1→4)-O-α-D-glucopyranosyl-(1→4)-α-D-glucopyranoside hereinafter referred to as "FG5P")

(1) Synthesis of mono-6-deoxy-6[(2-pyridyl)amino]-β-cyclodextrin (hereinafter referred to as "F-β-CD")

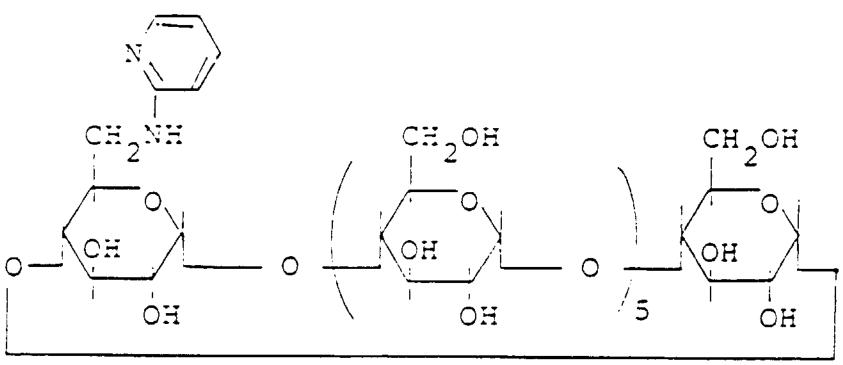

In 120 ml of dimethylsulfoxide (DMSO), 20 g of β-cyclodextrin and 30.4 g of dicyclohexylcarbodiimide (DCC) were dissolved and 2.6 ml of dichloroacetic acid was added thereto and stirred at room temperature for 30 minutes. To this, a solution obtained by dissolving 12.8 g of oxalic acid in 50 ml of methanol was added and the reaction solution was made pH 9 with 2 M aqueous solution of sodium carbonate. Further, a solution obtained by dissolving 1.6 g of 2-aminopyridine in 200 ml of water was added thereto, followed by addition of 3.6 g of pyridine borane to carry out the reaction at 65 - 70°C for 2 hours. Then, 700 ml of water was added to the reaction solution and an insoluble matter was removed by filtration. Then, the reaction solution was made pH 7 with HCl, followed by addition of 2.4 g of sodium boro-hydride to carry out the reaction at room temperature for 1 hour. HCl was added to the reaction solution to make the pH 3 and to decompose excess sodium borohydride, followed by addition of ammonia water to make the pH 7.0. After adding 2 liters of acetone, a deposited precipitate was filtered to yield 3.0 g of F-β-CD.

| Elementary analysis: $C_{47}H_{74}O_{34}N_2$ | | | | |
|---|---|---|---|---|
| | C(%) | H(%) | N(%) | O(%) |
| Calcd. | 46.61 | 6.16 | 2.31 | 44.92 |
| Found | 46.61 | 6.17 | 2.42 | 44.80 |

(2) Synthesis of FG5P

In 1 liter of 10 mM ammonium acetate buffer (pH 6.5), 10 g of F-β-CD and 10 g of p-nitrophenyl α-glucoside were dissolved, and 1000 kU of CGTase was added thereto and reacted at 37°C for 5 hours. The reaction solution was made pH 4.0 with acetic acid, and 20 kU of glucoamylase (derived from Rhizopus niveus) was added thereto and reacted at 37°C for 10 hours. After freeze-drying the reaction solution, purification was carried out by using a column (30 x 1500 mm) packed with Bio-Gel p-2 (mfd. by Bio-Rad Co.) equilibrated with 50 mM acetic acid to give 1.1 g of FG5P.

The same reaction and aftertreatment as mentioned above were conducted by using 10 g of p-nitrophenyl β-glucoside in place of 10 g of p-nitrophenyl α-glucoside to give 2.5 g of β-form FG5P.

The structure was identified by the method disclosed in Japanese Patent Unexamined Publication No. 61-83195.

Example 2

Synthesis of p-nitrophenyl O-(6-O-carboxymethyl)-α-D-glucopyranosyl-(1→4)-O-α-D-glucopyranosyl-(1→4)-O-α-D-glucopyranosyl-(1→4)-O-α-D-glucopyranosyl-(1-4)-α-D-glucopyranoside (hereinafter referred to as "CMG5P")

(1) Synthesis of mono-O-carboxymethyl-β-cyclodextrin (hereinafter referred to as "CM-β-CD")

In 120 ml of water, 15.0 g of β-cyclodextrin and 13.5 g of sodium hydroxide were dissolved and 90 ml of a 10% monochloroacetic acid aqueous solution was added thereto and stirred at 25°C for 5 hours. After making the reaction solution pH 7.0 with 6N HCl, 600 ml of acetone was added and deposited precipitate was filtered to give 12 g of CM-β-CD.

| Elementary analysis: $C_{44}H_{75}O_{37}N$, MW 1210 ($NH_4$ salt) | | | | |
|---|---|---|---|---|
| | C(%) | H(%) | O(%) | N(%) |
| Calcd. | 43.67 | 6.25 | 48.92 | 1.16 |
| Found | 43.66 | 6.28 | 48.86 | 1.20 |

(2) Synthesis of CMG5P

In 1 liter of 10 mM ammonium acetate buffer (pH 6.5), 10 g of CM-β-CD and 10 g of p-nitrophenyl-glucoside were dissolved, and 1000 kU of CGTase was added thereto and reacted at 37°C for 5 curs. Then, the pH was made 4.0 with acetic acid, and 20 kU of glucoamylase was added thereto and reacted at 37°C for 10 hours. After freeze-drying the reaction solution, purification was carried out by using a column (30 x 1500 mm) packed with Bio-Gel p-2 (mfd. by Bio-Rad Co.) equilibrated with 50 mM acetic acid to give 0.5 g of CMG5P together with 3 g of $C_2$ position substituted body and 1.7 g of $C_3$ position substituted body.

Example 3

Synthesis of phenyl O-(α-D-glucopyranosyl uronic-acid-(1→4)-O-α-D-glucopyranosyl-(1→4)-O-α-D-glucopyranosyl-(1→4)-O-α-D-glucopyranosyl-(1→4) α-D-glucopyranoside (hereinafter referred to as "carboxyl G5P")

(1) Synthesis of monocarboxyl-β-cyclodextrin (hereinafter referred to as carboxyl-β-CD)

In 1 liter of water, 50 g of β-cyclodextrin was dissolved, and 5 g of platina-carbon (10%) and 2.5 ml of isopropanol were added thereto. Then, air was introduced into the resulting solution at a flow rate of 50 ml/min with stirring at 70°C. While keeping the pH at neutral by adding 1 M sodium hydrogen carbonate dropwise, the reaction was carried out for 2.5 hours. After filtration, the filtrate was subjected to ion exchange chromatography (Dowex I) to yield 10.5 g of carboxyl-β-CD. Elementary analysis: $C_{42}H_{71}O_{36}N$, MW 1165 ($NH_4$ salt)

| Elementary analysis: $C_{42}H_{71}O_{36}N$, MW 1165 ($NH_4$ salt) | | | | |
|---|---|---|---|---|
| | C(%) | H(%) | O(%) | N(%) |
| Calcd. | 43.25 | 6.14 | 49.41 | 1.20 |
| Found | 43.30 | 6.16 | 49.40 | 1.14 |

(2) Synthesis of carboxyl-G5P

In 1 liter of 10 mM ammonium acetate buffer (pH 6.5), 10 g of carboxyl-β-CD and 10 g of phenyl α-glucoside were dissolved, and 1000 kU of CGTase was added thereto and reacted at 37°C for 1 hour. Then, the pH was made 4.0 with acetic acid, and 20 kU of glucoamylase was added thereto and the reaction was carried out at 37°C for 10 hours. After freeze-drying the reaction solution, purification was carried out by using a column (30 x 2400 mm) packed with Bio-Gel p-2 (mfd. by Bio-Rad Co.) equilibrated with 20 mM acetic acid to give 1.2 g of carboxyl-G5P.

The number of glucose residue per phenyl group in the resulting carboxyl-G5P was analyzed by gas-liquid chromatography (GLC) as follows.

G.L.C. analyses

Glucose residue and carboxyl glucose residue in carboxyl G5P were analyzed by G.L.C. (column, 2% OV-17 on Chromosorb W. (mfd. by Wako Pure Chemical Industries, Ltd., 0.4 x 200 cm) after methanolysis (1.4 M HCl-methanol, 90°C for 2 hours) followed by trimethylsilylation with hexamethyldisilazane and trimethylsilyl chloride in pyridine. The temperature was programmed from 110 to 250°C at the rate of 4°C increments per minute. The concentration of phenyl group was taken as unity, and the content was estimated from the absorbance at 265 nm in 0.1 M acetic acid solution. Further, the phenyl group was measured quantitatively from the absorbance at 265 nm in 0.1 M acetic acid. As a result, the ratio of glucose/phenol in the carboxyl-G5P was 3.9.

From the above results and the fact that the carboxyl-G5P was not acted by glucoamylase, the structure of carboxyl-G5P was estimated as follows:

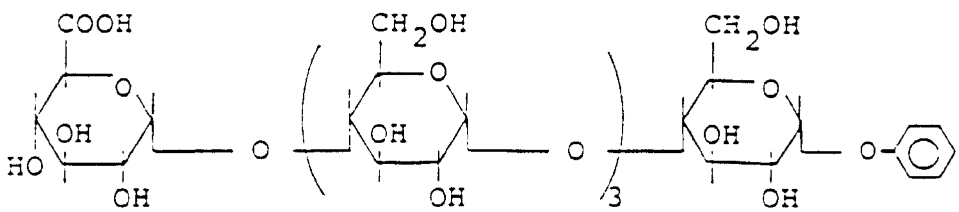

The mass spectrum of this compound is shown in Fig. 1.

Example 4

Synthesis of O-6-deoxy-6-[(2-pyridyl)amino]-α-D-glucopyranosyl-(1→4)-O-α-D-glucopyranosyl-(1→4)-O-α-D-glucopyranosyl-(1→4)-O-α-D-glucopyranosyl-(1→4)-O-α-D-glucopyranosyl-(1→4)-D-glucitol (hereinafter referred to as "FG6R")

In 1 liter of 10 mM ammonium acetate buffer (pH 6.5), 10 g of F-β-CD botained in the same manner as described in Example 1 (1), and 50 g of maltotriitol were dissolved, and 1000 kU of CGTase was added thereto. The reaction was carried out at 37°C for 5 hours. After the pH was made 4.0 with acetic acid, 20 kU of gluco-amylase was added to the reaction solution and the reaction was carried out at 37°C for 10 hours. After freeze-drying the reaction solution, purification was carried out by using a column (30 x 1500 mm) packed with Bio-Gel p-2 (mfd. by Bio-Rad Co.) equilibrated with 50 mM acetic acid to give 1.2 g of FG6R.

Example 5

Synthesis of phenyl O-(6-amino-6-deoxy)-α-D-gluco-pyranosyl-(1→4)-O-α-D-glycopyrancsyl-(1→4)-O-α-D-glycopyranozyl-(1→4)-O-α-D-glucopyranosyl-(1→4)-α-D-glucopyranoside (hereinafter referred to as "amino-G5P")

(1) Synthesis of mono-6-O-p-toluenesulfonyl β-cyclodextrin

In 100 ml of pyridine, 5.0 g of β-cyclodextrin was dissolved and 0.8 g of p-toluenesulfonyl chloride was added thereto, followed by stirring at 4°C for 15 hours. After concentration under reduced pressure, the reaction solution was charged in a column (5 x 60 cm) packed with activated carbon and eluted with water, 30% ethanol, 25% n-propanol, each in amounts of 3 liters. The 25% n-propanol fractions were collected and concentrated under reduced pressure to yield 0.7 g of 6-O-p-toluenesulfonyl β-cyclodextrin.

(2) Synthesis of mono-6-azide-6-deoxy-β-cyolodextrin

In 80 ml of water, 1.0 g of 6-O-p-toluenesulfonyl β-cyclodextrin was dissolved and 1 g of sodium azide was added thereto. The reaction was carried out at 95°C for 90 minutes. After the reaction, the reaction solution was concentrated under reduced pressure and separated and purified by gel filtration (Bio-Gel p-2) to yield 0.7 g of mono-6-azide-6-deoxy-β-cyclodextrin.

(3) Synthesis of amino-G5P

In 50 ml of 10 mM ammonium acetate buffer (pH 6.5), 0.5 g of mono-6-azide-6-deoxy-β-cyclodextrin and 0.5 g of phenyl α-D-glucopyranoside were dissolved and 50 kU of CGTase was added thereto. The reaction was carried out at 37°C for 2 hours. After the pH was made 4.0 with acetic acid, 1 kU of glucoamylase was added to the reaction solution, followed by reaction at 37°C for 10 hours. To the reaction solution, 0.1 g of 10% palladium-carbon was added and hydrogen gas was flowed into the reaction solution at 25°C for 8 hours with stirring. After freeze-drying the reaction solution, the reaction solution was separated and purified by gel filtration (Bio-Gel p-2) to give 80 mg of amino-G5P.

The number of glucose residue per phenyl group in the amino-G5P was measured by gas-liquid chromatography in the same manner as described in Example 3. As a result, the ratio of glucose/phenol in amino-G5P was 3.6.

From the results mentioned above and the fact that the amino-G5P was not acted by glucoamylase and the color development by ninhydrin was observed on a TLC plate, the structure of amino-G5P was estimated as follows:

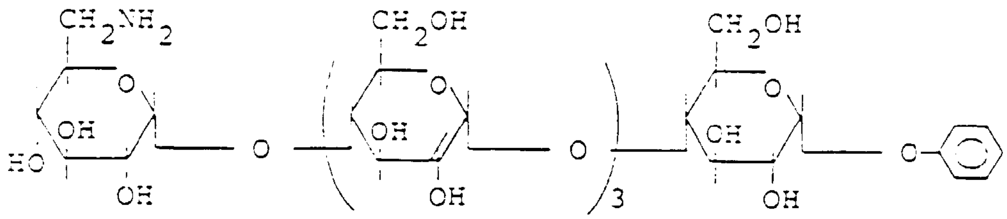

The mass spectrum of this compound is shown in Fig. 2.

Example 6

Synthesis of p-nitrophenyl O-(6-O-benzyl)-α-D-glycopyranosyl-(1→4)-O-α-D-glucopyranosyl-(1→4)-O-α-D-glucopyranosyl-(1→4)-O-α-D-glucopyranosyl-(1→4)-α-D-glucopyranoside (hereinafter referred to as "BG5P")

(1) Synthesis of mono-o-benzyl-β-cyclodextrin (hereinafter referred to as "benzyl-β-CD")

In 120 ml of water, 15 g of β-cyclodextrin and 13.5 g of sodium hydroxide were dissolved, and 15 ml of benzyl chloride was added thereto. The reaction was carried out at 10°C for 2 hours with stirring. After the reaction, the pH was made 7.0 with 6N HCl and 1 liter of acetone was added thereto. The deposited precipitate was filtered and 5 g of benzyl-β-CD was obtained (a mixture of substituted bodies having substituents at $C_2$, $C_3$, and $C_6$ positions).

(2) Synthesis of BG5P

In 1 liter of 10 mM ammonium acetate buffer (pH 6.5), 10 g of benzyl-β-CD and 10 g of p-nitrophenyl α-D-glucopyranoside were dissolved and 1000 kU of CGTase was added thereto. The reaction was carried out at 37°C for 5 hours with stirring. After making the pH 4.0 with acetic acid, 20 kU of glucoamylase was added thereto and the reaction was carried out at 37°C for 10 hours. After freeze-drying the reaction solution, purification was carried out by using a column (30 x 1500 mm) packed with Bio-Gel P-2 (mfd. by Bio-Rad Co.) equilibrated with 50 mM acetic acid to give 1.6 g of crude BG5P (a mixture of compounds having substituents at $C_2$, $C_3$, and $C_6$ positions). This was separated and purified by high performance liquid chromatography (HPLC) to give 180 mg of $C_6$ position substituted body (700 mg of $C_2$ position substituted body and 650 mg of $C_3$ position substituted body).

HPLC: content 96% [column: silica gel Z-ODS, $5C_{18}$ (a trade name, mfd. by Wako Pure Chemical Industries, Ltd., 10 x 300 mm); eluate: linear gradient of 10% $CH_3CN$ - 0.1% AcOH and 90% $CH_3CN$ - 0.1% AcOH, flow rate 3 ml/min, measured at 305 nm]

IR: as shown in Fig. 3.
$^1$H-NMR: as shown in Fig. 4.

G.L.C. analyses ... Glucose residue and benzyl glucose residue in BG5P were analyzed by G.L.C. (column, 2% OV-17 on chromosorb mfd. by Wako Pure Chem. Ind. Ltd., 0.4 x 200 cm) after methanolysis (1.4 M HCl-methanol,

90°C for 2h) followed by trimethylsilylation with hexamethyldisilazane and trimethylsilyl chloride in pyridine. The temperature was programmed from 110 to 250°C at the rate of 4°C increments per minute. The concentration of p-nitrophenyl group was taken as unity, and the content was estimated from the absorbance at 305 mm in 0.1 M acetic acid solution.

From the results mentioned above and the fact that BG5P was not acted by glucoamylase, the structure of BG5P was estimated as follows:

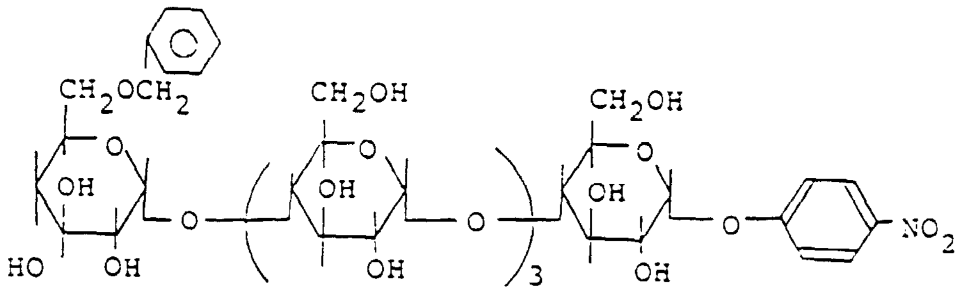

Example 7

Synthesis of p-nitrophenyl O-(6-o-methyl)-α-D-glycopyranosyl-(1→4)-O-α-D-glucopyranosyl-(1→4)-O-α-D-glucopyranosyl-(1→4)-O-α-D-glucopyranosyl-(1→4)-α-D-glucopyranoside (hereinafter referred to as "MG5P")

(1) Synthesis of mono-o-methyl-β-cyclodextrin (hereinafter referred to as "methyl-β-CD")

In 120 ml of water, 10 g of β-cyclodextrin and 13.5 g of sodium hydroxide were dissolved and 2.7 g of dimethyl sulfate was added thereto. The reaction was carried out at 10°C for 2 hours. After the reaction, the pH was made 7.0 with 6N HCl, and 1 liter of acetone was added to the reaction solution. The deposited precipitate was filtered to give 2 g of methyl-β-CD.

(2) Synthesis of MG5P

In 1 liter of 10 mM ammonium acetate buffer (pH 6.5), 10 g of methyl-β-CD and 10 g of p-nitrophenyl α-D-glucopyranoside were dissolved and 1000 kU of CGTase was added thereto. The reaction was carried out at 37°C for 5 hours with stirring. After making the pH 4.0 with acetic acid, 20 kU of glucoamylase was added to the reaction solution and the reaction was carried out at 37°C for 10 hours. After freeze-drying the reaction solution, purification was carried out by using a column (30 x 1500 mm) packed with Bio-Gel P-2 (mfd. by Bio-Rad Co.) equilibrated with 50 mM acetic acid to give 1.5 g of crude MG5P (a mixture of compounds having substituents at $C_2$, $C_3$, and $C_6$ positions). This was separated and purified by reverse high performance liquid chromatography (HPLC) to give 160 mg of $C_6$ position substituted body (680 mg of $C_2$ position substituted body and 560 mg of $C_6$ position substituted body).

HPLC: content 93% (measuring conditions were the same as described in Example 6).

The ratio of glucose/p-nitrophenol of MG5P obtained in the same manner as described in Example 6 was 3.9.

From the results mentioned above and the fact that MG5P was not acted by glucoamylase, the structure of MG5P was estimated as follows:

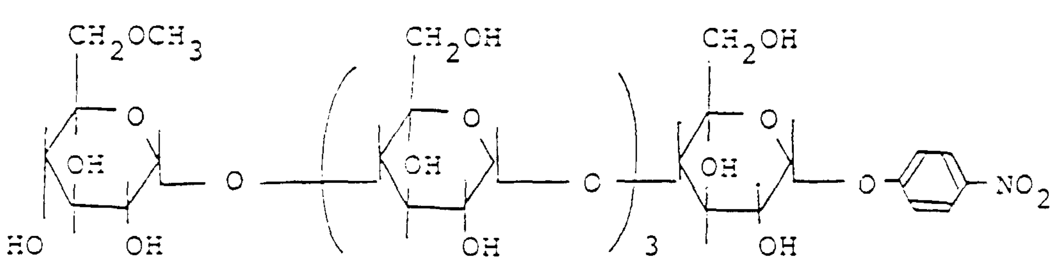

Example 8

Synthesis of p-nitrophenyl O-(6-bromo-6-deoxy)-α-D-glycopyranosyl-(1→4)-O-α-D-glucopyranosyl-(1→4)-O-α-D-glucopyranosyl-(1→4)-α-D-glucopyranoside (hereinafter referred to as "BrG5P")

(1) Synthesis of mono-6-bromo-6-deoxy-β-cyclodextrin (hereinafter referred to as "Br-β-CD")

In 250 ml of DMF, 5 g of mono-6-O-p-toluenesulfonyl β-cyclodextrin synthesized according to the process of Example 5(1) was dissolved and 15 g of lithium bromide was added thereto. The reaction was carried out for 2 hours with refluxing. After concentrating under reduced pressure, 500 ml of acetone was added thereto to deposite a precipitate, which was filtered to give 2.3 g of mono-6-bromo-6-deoxy-β-cyclodextrin.

(2) Synthesis of BrG5P

In 100 ml of 10 mM ammonium acetate buffer (pH 6.5), 1 g of Br-β-CD and 1 g of p-nitrophenyl α-D-glucopyranoside were dissolved and 100 kU of CGTase was added thereto. The reaction was carried out at 37°C for 5 hours with stirring. After making the pH 4.0 with acetic acid, 2000 U of glucoamylase was added thereto and the reaction was carried out at 37°C for 10 hours with stirring. The reaction solution was concentrated and purified by a column (30 x 1500 mm) packed with Bio-Gel P-2 (mfd. by Bio-Rad Co.) equilibrated with 50mM acetic acid to give 0.16 g of BrG5P.

HPLC: content 97% (measuring conditions were the same as described in Example 6)
$^1$H-NMR: as shown in Fig. 5.

The ratio of glucose/p-nitrophenol of BrG5P obtained in the same manner as described in Example 6 was 3.6.

From the results mentioned above and the fact that BrG5P was not acted by glucoamylase, the structure of BrG5P was estimated as follows:

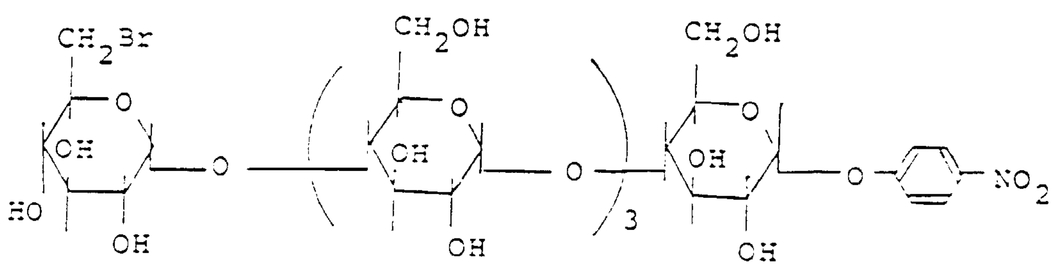

Example 9

Synthesis of BG5P

After dissolving 3 g of p-nitrophenyl-α-D-maltopentaoside in 50 ml of DMF, 15 ml of benzaldehyde dimethylacetal and 1 g of p-toluenesulfonic acid (anhydride) were added thereto. The reaction was carried out at 50°C for 3 hours with stirring. After the reaction, 100 ml of pyridine was added thereto, and 60 ml of benzoyl chloride was added dropwise to carry out the reaction. The reaction solution was poured into a solution of saturated sodium hydrogen carbonate to stop the reaction. The reaction solution was extracted with 200 ml of chloroform and washed with saturated saline solution twice, and the solvent was removed in vacuo. The resulting syrup was transferred to a 1-liter four-necked·flask and dissolved in 250 ml of THF, followed by addition and dissolution of 3 g of dimethylamine borane. To this solution, 10 ml of *a* solution of 0.5 M HCl (gas) in diethyl ether was added dropwise with stirring, and the reaction solution was poured into 200 ml of saturated sodium hydrogen carbonate to stop the reaction, followed by extraction with 200 ml of chloroform. After washing twice with saturated saline solution, the solvent was removed in vacuo. To the resulting solution, 500 ml of methanol solution containing 0.1N sodium methoxide was added and stirred at 25°C for 4 hours, followed by addition of acetic acid for neutralization. After removing the solvent in vacuo, 20 ml of 50 mM acetic acid solution was added and applied to a Bio-Gel p-2 column (mfd. by Bio-Rad Co., 2 cm in diameter x 150 cm) equilibrated with 50 mM acetic acid and the 3G5P fractions were collected and freeze-dried.

| | |
|---|---|
| Yield | 450 mg |
| IR chart | as shown in Fig. 3 |
| H-NMR | as shown in Fig. 4 |
| HPLC | content 95% (measuring conditions |

were the same as described in Example 6)

GC composition analysis: glucose/6-0-benzyl-glucose/p-nitrophenyl group = 3.8/0.9/1.0 (measuring conditions were the same as described in Example 6)

Example 10

Synthesis of p-nitrophenyl O-(6-O-benzyl)-α-D-glycopyranosyl-(1→4)-O-α-D-glucopyranosyl-(1→4)-O-α-D-glucopyranosyl-(1→4)-O-α-D-glucopyranosyl-(1→4)-O-α-D-glucopyranosyl-(1→4)-α-D-glucopyranoside (hereinafter referred to as "BG6P")

After dissolving 3 g of p-nitrophenyl α-D-maltohexaoside in 50 ml of DMF, 15 ml of benzaldehyde dimethylacetal and 1 g of p-toluenesulfonic acid (anhydride) were added thereto and reacted at 50°C for 3 hours with stirring. After the reaction, 100 ml of pyridine was added thereto and the reaction was carried out while adding 80 ml of benzoyl chloride dropwise with ice cooling. The reaction solution was poured into 300 ml of saturated solution of sodium hydrogen carbonate to stop the reaction. After extracting with 200 ml of chloroform and washing with saturated saline solutlon twice, the solvent was removed in vacuo. The resulting syrup was transferred to a 1-liter four-necked flask and dissolved in 250 ml of THF, followed by addition and dissolution of 3 g of dimethylamine borane. To this solution, 10 ml of a 0.5 M HCl (gas) in diethyl ether was added dropwise with stirring. The reaction solution was poured into 300 ml of a solution of saturated sodium hydrogen carbonate to stop the reaction, followed by extraction with 200 ml of chloroform. After washing with saturated saline solution twice and removing the solvent in vacuo, 500 ml of a methanol solution containing 0.1N sodium methoxide was added thereto, followed by stirring at 25°C for 4 hours. Then, acetic acid was added for neutralization. After removing the solvent in vacuo, 20 ml of 50 mM acetic acid solution was added thereto and applied to a Bio Gel P-2 column (mfd. by Bio-Rad Co., 2 cm in diameter x 150 cm) equilibrated with 50 mM acetic acid solution. The BG6P fractions were collected and freeze-dried.

| | |
|---|---|
| Yield: | 500 mg |
| HPLC: | content 94% (measuring conditions were the same as Example 6) |

GC composition analysis: glucose/6-O-benzyl-glucose/p-nitrophenyl group = 4.9/0.9/1.0 (measuring conditions were the same as described in Example 6)

Example 11

Synthesis of MG5P

After dissolving 5 g of p-nitrophenyl α-D-maltopentaoside in 40 ml of DMF, 50 ml of methylal and 1 g of p-toluenesulfonic acid (anhydride) were added and reacted at 50°C for 3 hours. After concentrating the reaction solution in vacuo, 300 ml of pyridine and 100 ml of acetic anhydride were added and reacted at room temperature for 12 hours. Then, 500 ml of a solution of saturated sodium hydrogen carbonate was added to the reaction solution to stop the reaction. After extracting by adding 100 ml of chloroform, washing with saturated saline solution was conducted twice, followed by removal of the solvent by distillation. The resulting syrup was transferred to a 1-liter four-necked flask and dissolved in 250 ml of THF, followed by addition and disslution of 3 g of dimethylamine borane. To this solution, 10 ml of a 0.5 M HCl (gas) in diethyl ether was added dropwise with stirring. The resulting syrup was transferred to a 1-liter four-necked flask and dissolved in 250 ml of THF, followed by addition and dissolution of 3 g of dimethylamine borane. To this solution, 10 ml of a 0.5 M HCl (gas) in diethyl ether was added dropwise with stirring. To this, 700 ml of methanol solution containing 0.1 N sodium methoxide was added and reacted at 25°C for 4 hours. Then, acetic acid was added for neutralization. After removing the solvent in vacuo, the resulting solution was applyed to a Bio-Gel P-2 column (mfd. by Bio-Rad Co., 2 cm in diameter x 150 cm) equilibrated with 50 mM acetic acid. The MG5P fractions were collected and freeze-dried.

| | |
|---|---|
| Yield: | 730 mg |

HPLC: content 93% (measuring conditions were the same as Example 6)

Example 12

Measurement of α-amylase activity

[Measuring reagent]

A measuring reagent solution was prepared by dissolving 30 mg of BG5P obtained in Example 6 in 30 ml of 50 mmol/l 2-(N-morpholino)ethanesulfonic acid (MES)-NaOH buffer (pH 6.9) containing 400 units of glucoamylase, 300 units of α-glucosidase and 20 mmol/l of calcium chloride.

[Measuring procedure]

To 2 ml of the measuring solution, 100 μl of a sample serum was added and incubated at 37°C. The change of absorbance of the reaction solution at a wavelength of 405 nm was measured.

On the other hand, using a standard sample containing known activities of α-amylase, the calibration curve was prepared in the same manner as mentioned above. From this calibration curve, α-amylase activity of a sample serum was obtained. Fig. 6 shows a relationship between the α-amylase activity at individual diluted stages of a standard sample (Somogyi unit/dl) and the increase in absorbance (ΔA) per minute at a wavelength of 405 nm.

As is clear from Fig. 6, the calibration curve obtained by lining the plots of increased amounts of absorbance (ΔA/min) corresponding to α-amylase activity (Somogyi unit/dl) is linear and passing through the zero point. This means that the calibration curve shows good quantitativeness.

Example 13

Measurement of α-amylase activity

[Measuring reagent]

A measuring reagent solution was prepared by dissolving 2.1 g of N,N-bis(2-hydroxyethyl)-2-amino-ethanesulfonic acid, 230 mg of NaCl, 58 mg of $CaCl_2$, 500 mg of NaOH in distilled water, making the total amount 100 ml (pH 7.3) and dissolving 125 mg of BG5P obtained in Example 9 therein.

Solution Ⓐ: To 50 ml of the resulting solution, 25,000 units of α-glucosidase was added.

Solution Ⓑ: To 20 ml of the solution Ⓐ, 1000 units of glucoamylase was added.

[Measuring procedure]

To 2 ml of the reagent solution Ⓐ or Ⓑ, 50 μl of serum sample was added and incubated at 37°C. The change of absorbance of the reaction solution at a wavelength of 405 nm was measured.

Increasing amounts of absorbances (ΔA) per minute when measured by using 5 kinds of human serum at a wavelength of 405 nm were shown in Table 4.

Table 4

| Human serum No. | Reagent solution | |
|---|---|---|
| | Ⓐ | Ⓑ |
| 1 | 0.0330 | 0.0335 |
| 2 | 0.0291 | 0.0291 |
| 3 | 0.0329 | 0.0330 |
| 4 | 0.0535 | 0.0537 |
| 5 | 0.0886 | 0.0885 |

As is clear from Table 4, the measuring sensitivity is hardly influenced by the addition of glucoamylase.

Example 14

Measurement of α-amylase activity

[Measureing reagent]

A measuring reagent solution was prepared by dissolving 30 mg of BrG5P obtained in Example 8 in 30 ml of 50 mmol/l MES-NaOH buffer (pH 6.9) containing 400 units of glucoamylase, 300 units of α-glucosidase and 20 mmol/l of calcium chloride.

[Measuring procedure]

To 2 ml of the measuring solution, 100 μl of a serum sample was added and incubated at 37°C. The change of absorbance of the reaction solution at a wavelength of 405 nm was measured.

On the other hand, using a standard sample containing known activities of α-amylase, the calibration curve was prepared in the same manner as mentioned above. From this calibration curve, α-amylase activity was obtained. Fig. 7 shows a relationship between the α-amylase activity at individual diluted stages of a standard sample (Somogyi unit/dl) and the increase in absorbance (ΔA) per minute at a wavelength of 405 nm.

As is clear from Fig. 7, the calibration curve obtained by lining the plots of increased amounts of absorbance (ΔA/min) corresponding to α-amylase activity (Somogyi unit/dl) is linear and passing through the zero point. This means that the calibration curve shows good quantitativeness.

## Claims

**1.** A process for producing a compound of the formula:

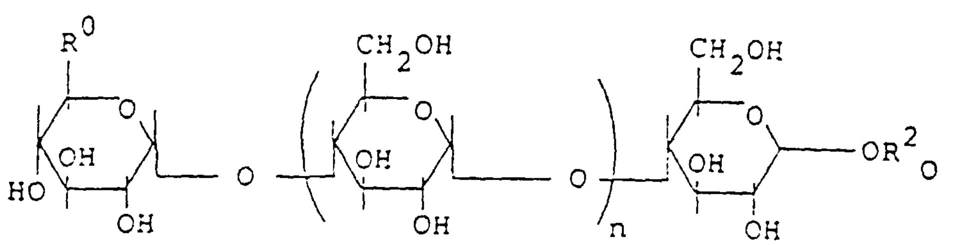 I

wherein

$R^0$ is an alkoxymethyl group, a benzyloxymethyl group, an aminomethyl group, a carboxyl group, a halomethyl group, a pyridylaminomethyl group, or a carboxymethoxymethyl group,
$R^2_O$ is glucitol residue or a group of the formula:

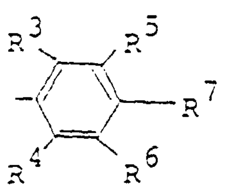 , 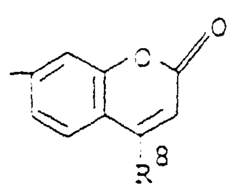 or 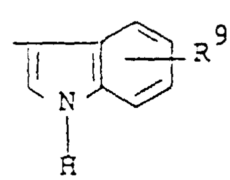

in which $R^3$ through $R^6$ are independently hydrogen, a lower alkyl group, a lower alkoxy group, a nitro group, a carboxyl group, a sulfonic acid group or a halogen atom, and $R^3$ and $R^5$, and/or $R^4$ and $R^6$, may be bonded to form an aromatic ring; $R^7$ is hydrogen, a lower alkoxy group, a halogen atom, or a nitro group; $R^8$ is hydrogen, a methyl group, or a trifluoromethyl group; and $R^9$ is hydrogen or a halogen atom; and n is an integer of 2 to 5, which comprises reacting a modified cyclodextrin with cyclomaltodextrin-glucanotransferase in the presence of an acceptor selected from derivatives of glucose, maltose and maltotriose wherein the hydroxyl group at

the $C_1$ position of the acceptor is replaced by a substituent represented by the formula: $-OR^2_O$, wherein $R^2_O$ is as defined above and then reacting with glucoamylase or α-glucosidase.

**2.** A process for producing a compound of the formula:

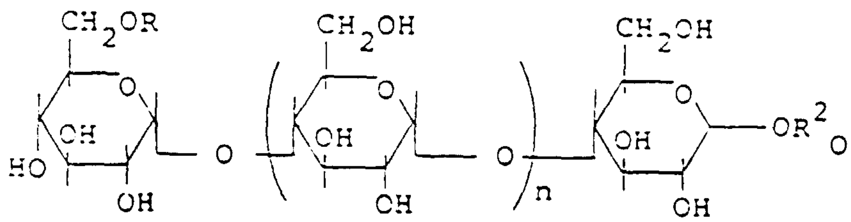

II

wherein R is a benzyl group, a substituted benzyl group, a 2-, 3- or 4-pyridylmethyl group, a straight- or branched-chain or cyclic alkyl group having 1 to 6 carbon atoms, or an alkenyl group having 1 to 6 carbon atoms; wherein the substituent to said substituted benzyl group is an alkyl group having 1 to 4 carbon atoms, an alkoxy group having 1-4 carbon atoms, an alkyl substituted amino group, a carboxyl group, a nitro group or a halogen group; $R^2_O$ is a glucitol residue or a group of the formula:

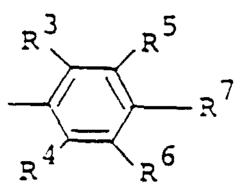

,

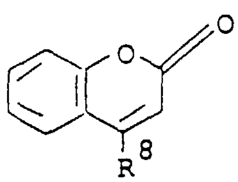

or

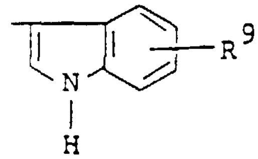

in which $R^3$ through $R^6$ are independently hydrogen, a lower alkyl group, a lower alkoxy group, a nitro group, a carboxyl group, a sulfonic acid group or a halogen atom, and $R^3$ and $R^5$, and/or $R^4$ and $R^6$, may be bonded to form an aromatic ring; $R^7$ is hydrogen, a lower alkoxy group, a halogen atom, or a nitro group; $R^8$ is hydrogen, a methyl group, or a trifluoromethyl group; and $R^9$ is hydrogen or a halogen atom; and n is an integer of 2 to 5, which comprises reacting an oligosaccharide having a substituent which exhibits an optically measurable change upon cleavage at the reducing-end glucose residue with an aldehyde, a ketone, an acetal or a ketal to form a 4,6-0-cyclic acetal or a 4,6-0-cyclic ketal at the non-reducing-end glucose residue, and reducing said 4,6-O-cyclic acetal or ketal.

**3.** A process according to Claim 2, wherein the oligosacoharide used as a starting material is represented by the formula:

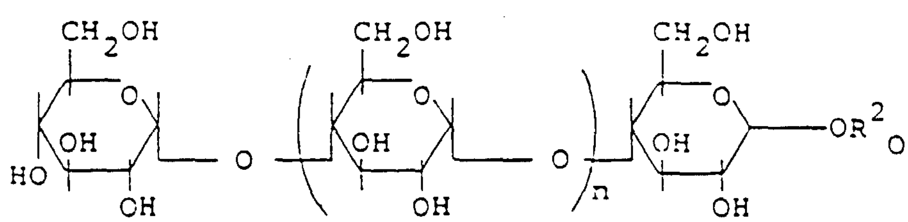

(VIII)

wherein $R^2_O$ and n are as defined in Claim 2.

**Patentansprüche**

1. Verfahren zum Herstellen einer Verbindung der Formel:

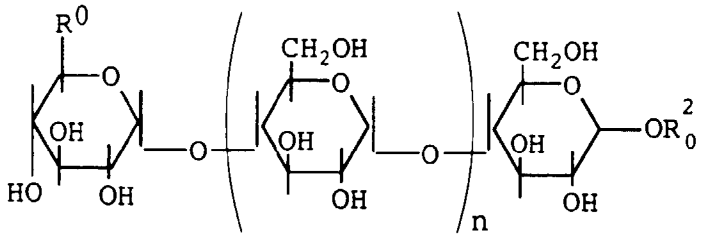

I

worin $R^0$ eine Alkoxymethyl-Gruppe, eine Benzoyloxymethyl-Gruppe, eine Aminomethyl-Gruppe, eine Carboxyl-Gruppe, eine Halogenmethyl-Gruppe, eine Pyridylaminomethyl-Gruppe oder eine Carboxymethoxymethyl-Gruppe ist, $R_O^2$ ist ein Sorbit-Rest oder eine Gruppe der Formel:

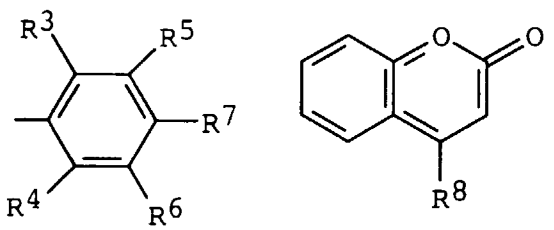

oder

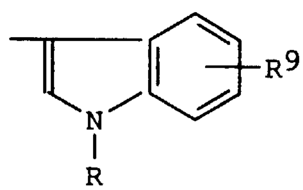

worin $R^3$ bis $R^6$ unabhängig Wasserstoff, eine niedere Alkyl-Gruppe, eine niedere Alkoxy-Gruppe, eine Nitro-Gruppe, eine Carboxyl-Gruppe, eine Sulfonsäure-Gruppe oder ein Halogenatom sind; und $R^3$ und $R^5$ und/oder $R^4$ und $R^6$ können unter Bildung eines aromatischen Ringes gebunden sein; $R^7$ ist Wasserstoff, eine niedere Alkoxy-Gruppe, ein Halogenatom oder eine Nitro-Gruppe; $R^8$ ist Wasserstoff, eine Methyl-Gruppe oder eine Trifluormethyl-Gruppe; und $R^9$ ist Wasserstoff oder ein Halogenatom; und n ist eine ganze Zahl von 2 bis 5; welches Verfahren das Umsetzen eines modifizierten Cyclodextrins mit Cyclomaltodextrin-Glucanotransferase in Gegenwart eines Akzeptors umfaßt, ausgewählt aus Derivaten von Glucose, Maltose und Maltotriose, worin die Hydroxyl-Gruppe in der Stellung $C_1$ des Akzeptors ersetzt wird durch einen Substituenten, dargestellt durch die Formel: $-OR_O^2$, worin $R_O^2$ wie vorstehend festgelegt ist; und nachfolgendes Umsetzen mit Glucoamylase oder α-Glucosidase.

2. Verfahren zum Herstellen einer Verbindung der Formel:

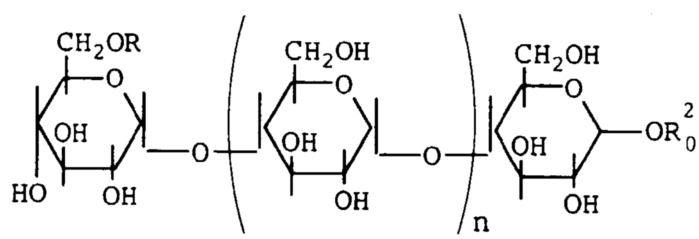

II

worin R eine Benzyl-Gruppe, eine substituierte Benzyl-Gruppe, eine 2-, 3- oder 4-Pyridylmethyl-Gruppe, eine geradkettige oder verzweigte oder cyclische Alkyl-Gruppe mit 1 bis 6 Kohlenstoffatomen oder eine Alkenyl-Gruppe mit 1 bis 6 Kohlenstoffatomen, wobei der Substituent an der substituierten Benzyl-Gruppe eine Alkyl-Gruppe mit 1 bis 4 Kohlenstoffatomen, eine Alkoxy-Gruppe mit 1 bis 4 Kohlenstoffatomen, eine Alkyl-substituierte Amino-Gruppe, eine Carboxyl-Gruppe, eine Nitro-Gruppe oder eine Halogen-Gruppe; $R_0^2$ ist ein Sorbit-Rest oder Gruppe der Formel:

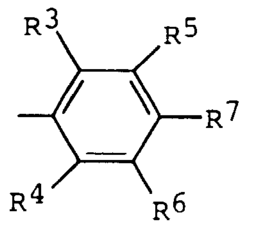

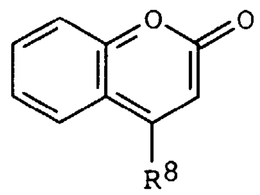

oder

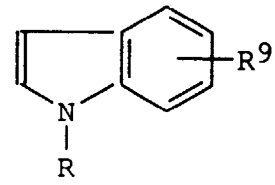

worin $R^3$ bid $R^6$ unabhängig Wasserstoff, eine niedere Alkyl-Gruppe, eine niedere Alkoxy-Gruppe, eine Nitro-Gruppe, eine Carboxyl-Gruppe, eine Sulfonsäure-Gruppe oder ein Halogenatom sind; und $R^3$ und $R^5$ und/oder $R^4$ und $R^6$ können unter Bildung eines aromatischen Ringes gebunden sein; $R^7$ ist Wasserstoff, eine niedere Alkoxy-Gruppe, ein Halogenatom oder eine Nitro-Gruppe; $R^8$ ist Wasserstoff, eine Methyl-Gruppe oder eine Trifluormethyl-Gruppe; und $R^9$ ist Wasserstoff oder ein Halogenatom; und n ist eine ganze Zahl von 2 bis 5; welches Verfahren das Umsetzen eines Oligosaccharids, das einen Substituenten aufweist, der beim Abspalten an dem reduzierenden Ende des Glucose-Restes eine optisch meßbare Änderung zeigt, mit einem Aldehyd, einem Keton, einem Acetal oder einem Ketal an dem nichtreduzierenden Ende des Glucose-Restes und das Reduzieren des 4,6-O-cyclischen Acetals oder Ketals umfaßt.

3. Verfahren nach Anspruch 2, bei welchem das als Ausgangsmaterial verwendete Oligosaccharid dargestellt wird durch die Formel:

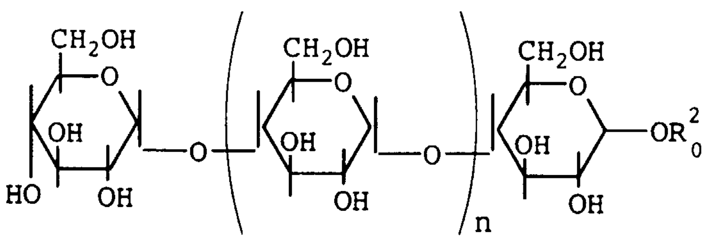

VIII

worin $R_O^2$ und n wie in Anspruch 2 festgelegt ist.

## Revendications

1. Procédé pour la production d'un composé de formule :

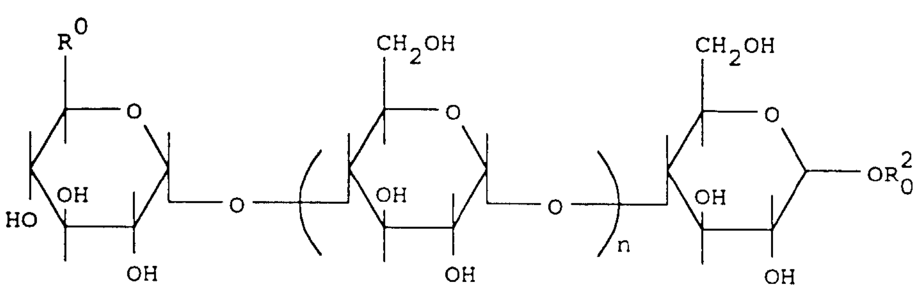

(I)

dans laquelle $R^0$ est un groupe alkoxyméthyle, benzyloxyméthyle, aminométhyle, carboxyle, halogénométhyle, pyridylaminométhyle, ou carboxyméthoxyméthyle ; $R_O^2$ est le résidu de glucitol ou un groupe de formule :

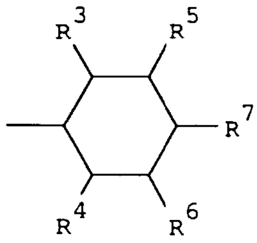 , 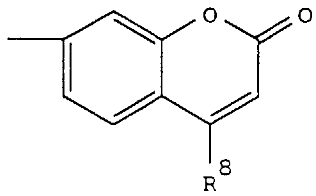 ou 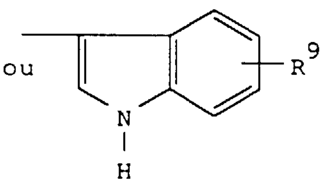

où $R^3$ à $R^6$ représentent indépendamment H, un groupe alkyle inférieur, alkoxy inférieur, nitro, carboxyle, acide sulfonique ou un atome d'halogène, $R^3$ et $R^5$, et/ou $R^4$ et $R^6$, pouvant être liés pour former un cycle aromatique, $R^7$ est H, alkoxy inférieur, nitro ou un atome d'halogène, $R^8$ est H, méthyle ou trifluoroiiiéthyle, et $R^9$ est H ou un atome d'halogène ; et n est un nombre entier ayant pour valeur 2 à 5, ledit procédé comprenant la réaction d'une cyclodextrine modifiée avec une cyclomaltodextrine-glucanotransférase en présence d'un accepteur choisi parmi les dérivés de glucose, maltose et maltotriose, le groupe hydroxyle en position $C_1$ de l'accepteur étant remplacé par un substituant représenté par la formule : $-OR_0^2$ où $R_0^2$ est défini comme indiqué ci-dessus, puis la réaction avec une glucoamylase ou une α-glucosidase.

**2.** Procédé pour la production d'un composé de formule

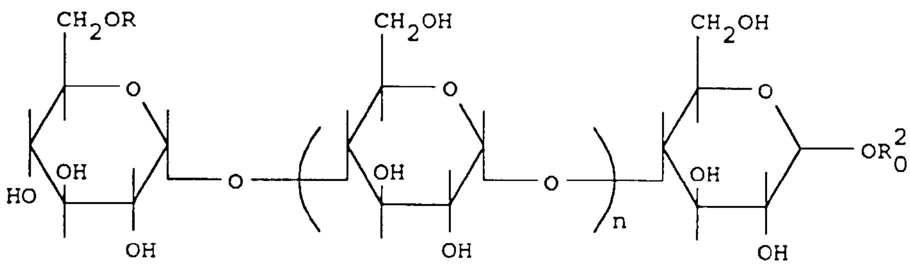 (II)

dans laquelle R est un groupe benzyle, benzyle substitué, 2-, 3- ou 4-pyridylméthyle, un groupe alkyle à chaîne droite ou ramifiée ou un groupe alkyle cyclique ayant de 1 à 6 atomes de carbone, ou un groupe alkényle ayant de 1 à 6 atomes de carbone, où le substituant dudit groupe benzyle substitué est un groupe alkyle ayant de 1 à 4 atomes de carbone, un groupe alkoxy ayant de 1 à 4 atomes de carbone, un groupe amino alkyle substitué, un groupe nitro ou un atonie d'halogène ; $R_0^2$ est le résidu de glucitol ou un groupe de formule :

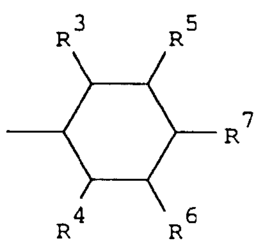 , 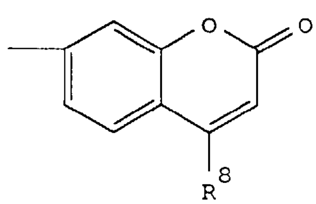 ou 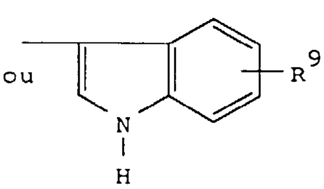

où $R^3$ à $R^6$ représentent indépendamment H, un groupe alkyle inférieur, alkoxy inférieur, nitro, carboxyle, acide sulfonique ou un atome d'halogène, $R^3$ et $R^5$, et/ou $R^4$ et $R^6$, pouvant être liés pour former un cycle aromatique, $R^7$ est H, alkoxy inférieur, nitro ou un atome d'halogène, $R^8$ est H, méthyle ou trifliioroiiiéthyle, et $R^9$ est H ou un atome d'halogène ; et n est un nombre entier ayant pour valeur 2 à 5, ledit procédé comprenant la réaction d'un oligosaccharide ayant un susbstituant qui présente un changement optiquement mesurable par clivage au niveau du reste glucose de l'extrémité réductrice avec un aldéhyde, une cétone, un acétal ou un cétal pour former un acétal 4,6-O-cyclique ou un cétal 4,6-O-cyclique au niveau du reste glucose de l'extrémité réductrice, et la réduction dudit acétal ou cétal 4,6-O-cyclique.

**3.** Procédé suivant la revendication 2, dans lequel l'oligosaccharide utilisé comme matière de départ est représenté par la formule :

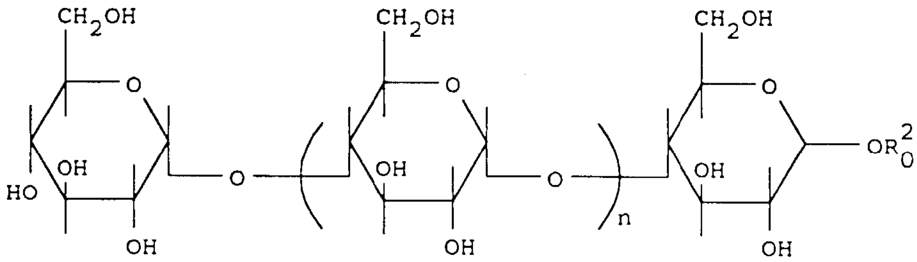

(VIII)

dans laquelle $R^2_O$ et n sont définis comme indiqué dans la revendication 2.

FIG. 1

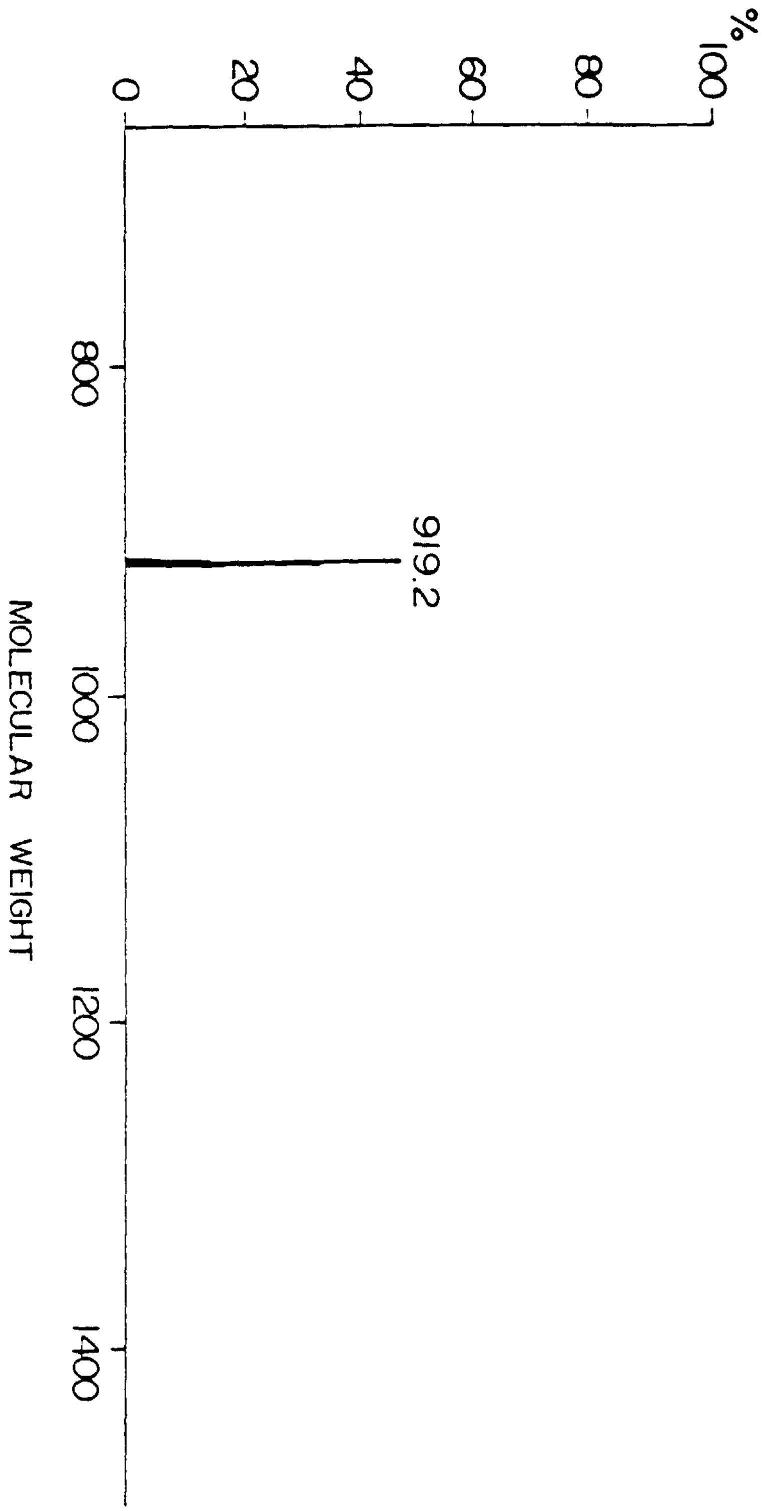

FIG. 2

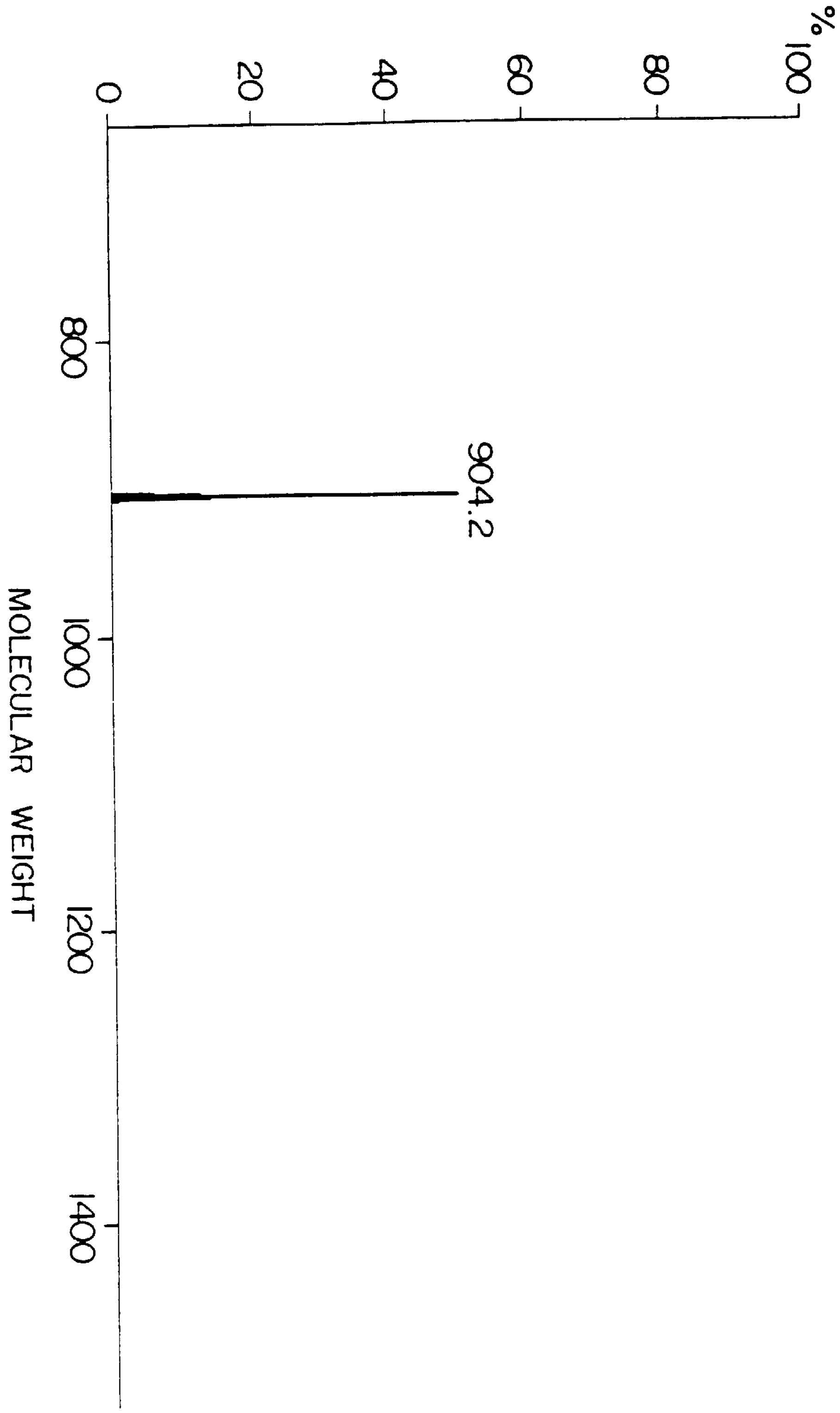

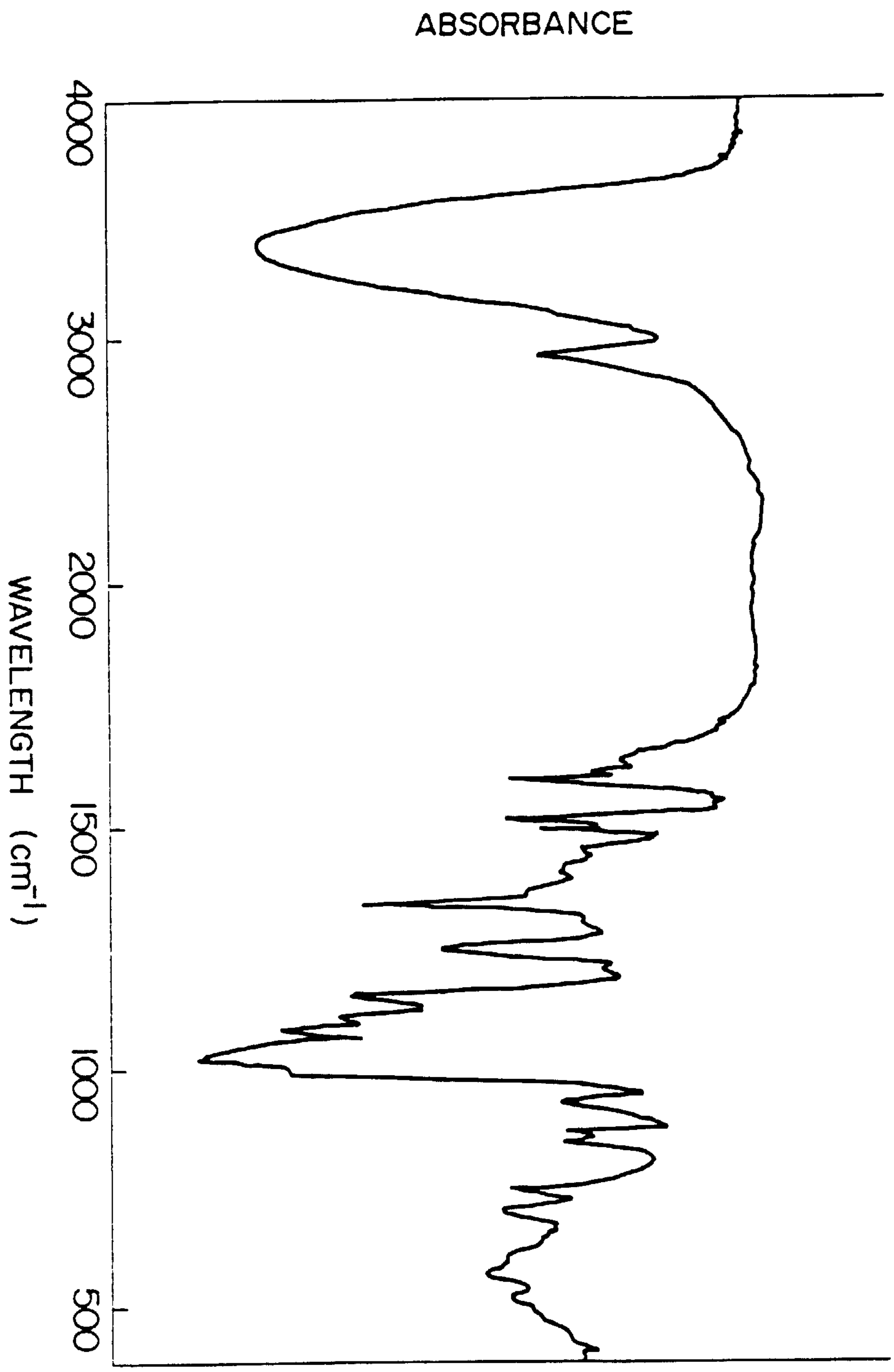


FIG. 3

FIG. 4

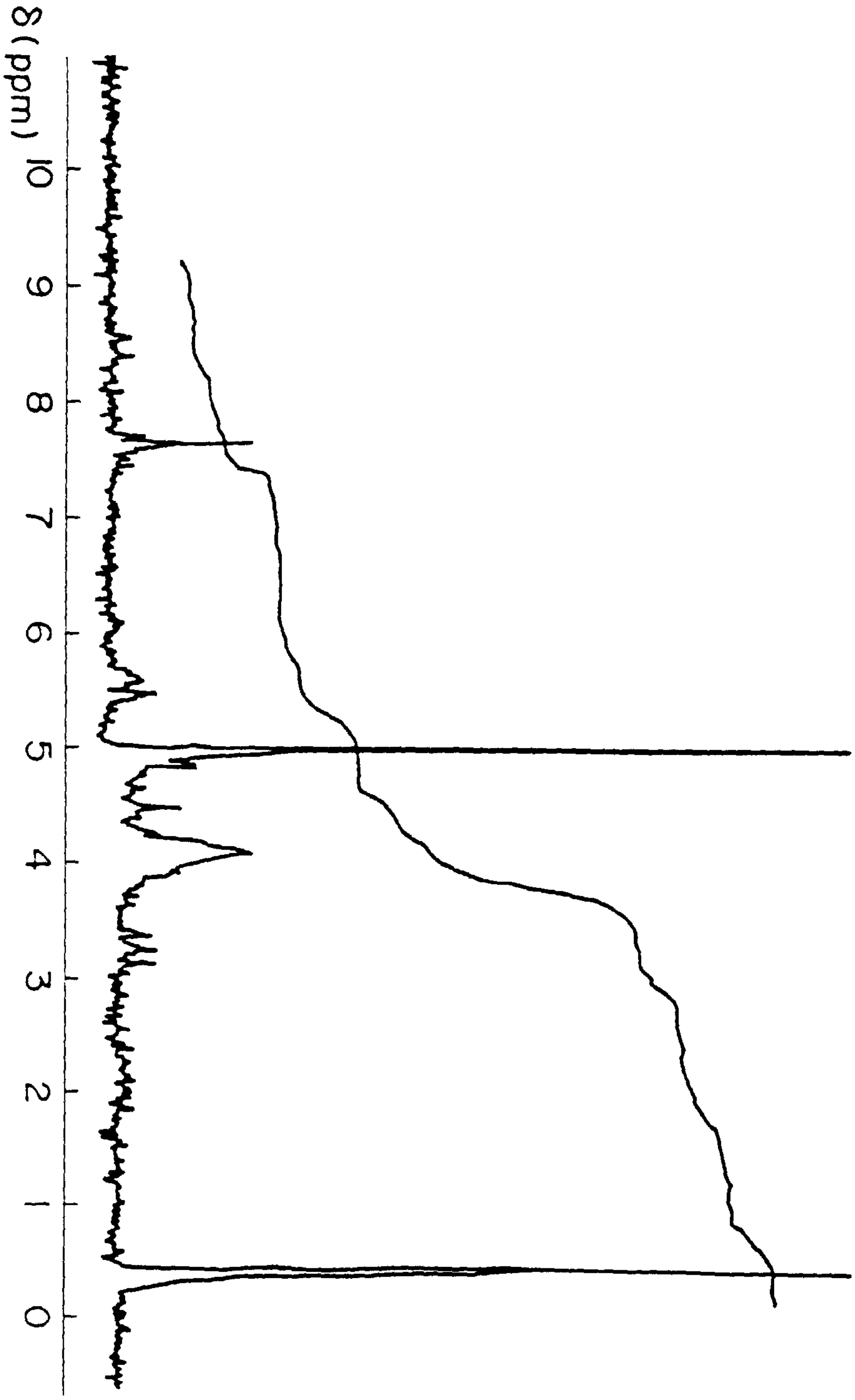

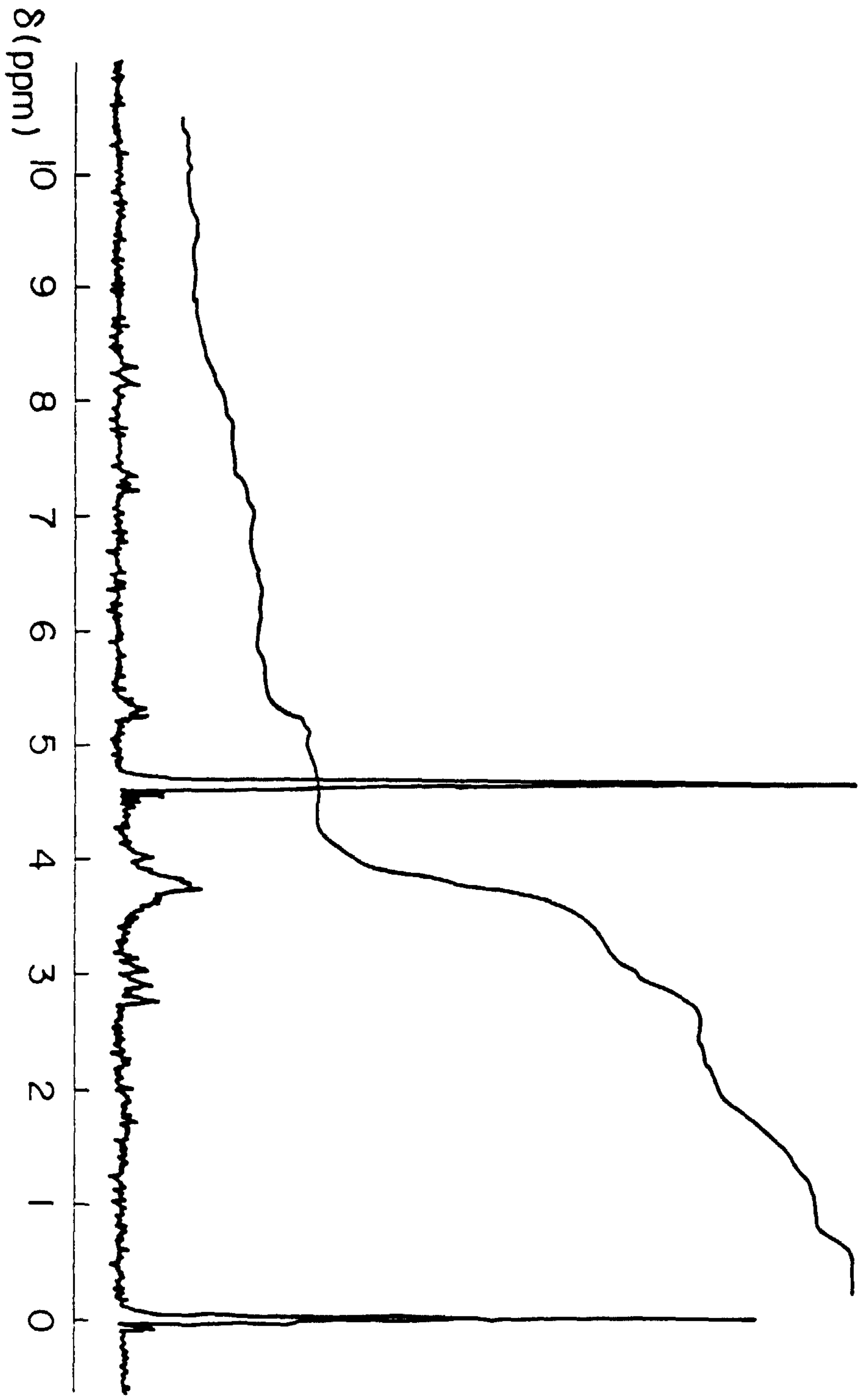


FIG. 5

FIG. 6

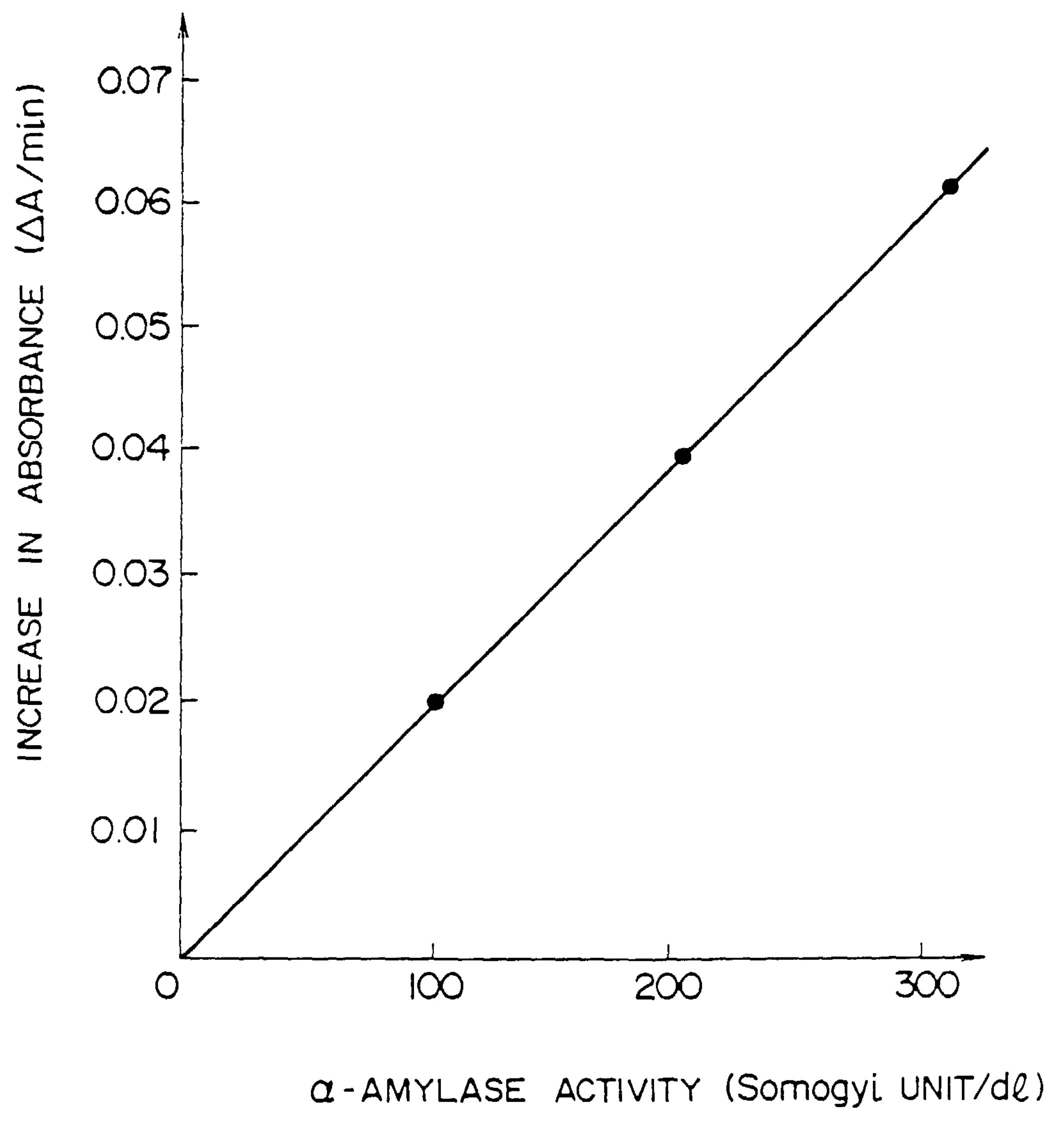

INCREASE IN ABSORBANCE (ΔA/min)
0.07
0.06
0.05
0.04
0.03
0.02
0.01
0
100
200
300
α-AMYLASE ACTIVITY (Somogyi UNIT/dℓ)

FIG. 7

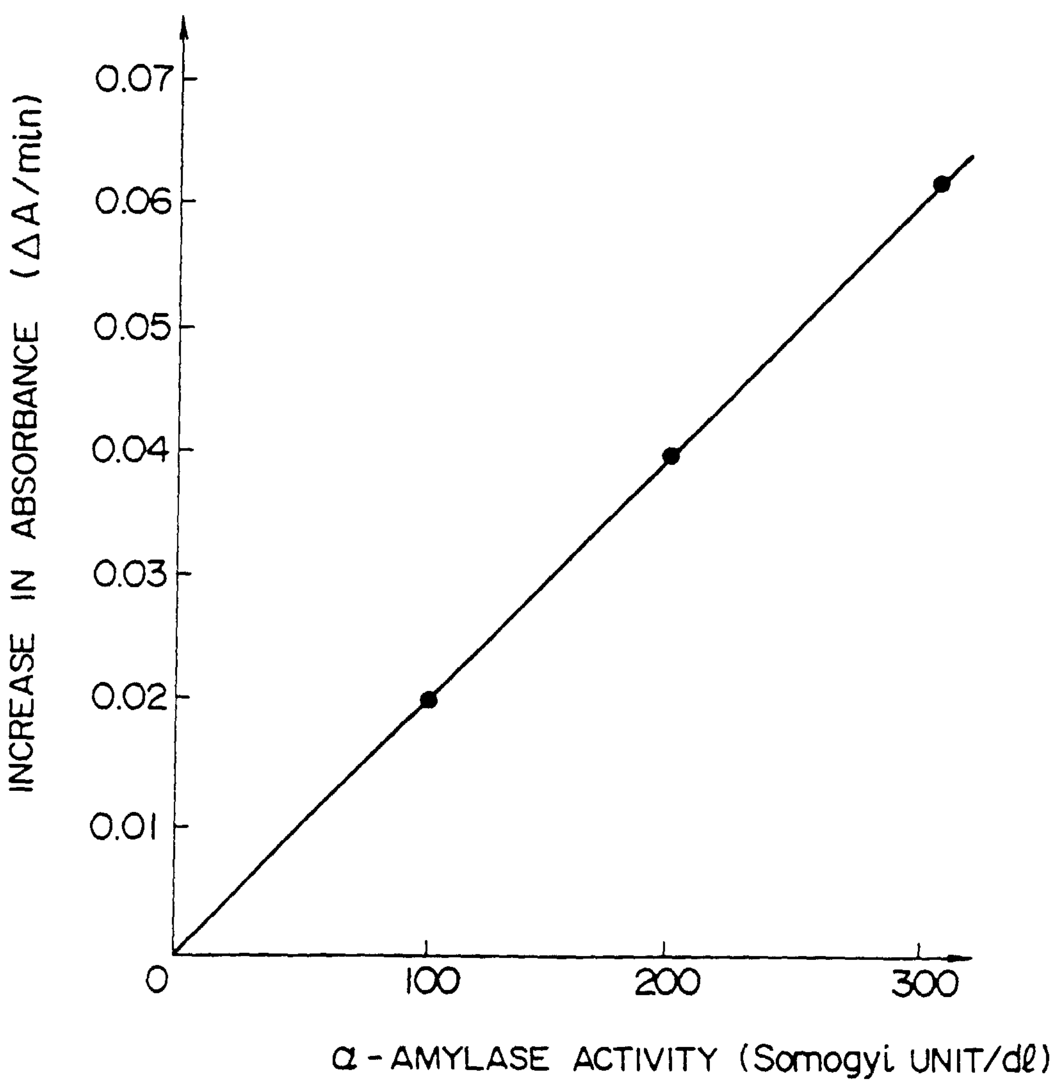
0.07
0.06
0.05
0.04
0.03
0.02
0.01
0
100
200
300
INCREASE IN ABSORBANCE (ΔA/min)
α-AMYLASE ACTIVITY (Somogyi UNIT/dℓ)